# EUROPÄISCHE PATENTSCHRIFT

(11) **EP 1 725 177 B1**
(45) Veröffentlichungstag und Bekanntmachung des Hinweises auf die Patenterteilung: **02.11.2011**
(21) Anmeldenummer: 05702292.3
(22) Anmeldetag: 19.01.2005
(51) Int. Cl.: A61B 17/72, A61B 17/88

(54) **REPOSITIONSWERKZEUG**
RESETTING TOOL
OUTIL DE REPOSITIONNEMENT

(30) Priorität: 11.03.2004 WO PCT/IB2004/000677
(43) Veröffentlichungstag der Anmeldung: 29.11.2006
(73) Patentinhaber: Synthes GmbH, 4436 Oberdorf (CH)
(72) Erfinder: SCHWER, Stefan, 79540 Lörrach (CH); BAPST, Reto, 6010 Kriens (CH)
(74) Vertreter: Höhfeld, Jochen
(86) Internationale Anmeldenummer: PCT/IB2005/000130
(87) Internationale Veröffentlichungsnummer: WO 2005/092225

(56) Entgegenhaltungen:
- DE-A- 4 418 974
- US-A- 5 458 604
- US-A- 5 993 450
- US-B1- 6 402 757

## Beschreibung

Die Erfindung betrifft ein Repositionswerkzeug und eine Mutter für ein Repositionswerkzeug zum axialen Verschieben eines Kirschner-Drahtes gemäss dem Oberbegriff der Ansprüche 1, 11 und 15.

Kirschner-Drähte werden bei der Fixierung von Frakturen häufig eingesetzt. Oftmals dienen sie dabei lediglich der vorübergehenden Fixierung und Ruhigstellung, während die für die Knochenheilung notwendige dauerhafte Fixierung beispielsweise durch Knochenschrauben sichergestellt wird. Ferner werden sie bei der Reposition der Fraktur eingesetzt. In diesen Fällen müssen die Kirschner-Drähte nach der Versorgung entfernt werden.

Ein Beispiel einer solchen Frakturversorgung sei anhand des gering invasiven Stabilisationssystems (LISS: Less Invasive Stabilization Systems) erläutert. Kennzeichen dieses Systems ist, dass ein plattenähnliches Implantat zusammen mit Verriegelungsschrauben als so genannter Fixateur interne agiert. Vorteil des Systems ist, dass die Blutversorgung des Knochens unter der Platte erhalten bleibt, da zwischen dem Plattenimplantat und Knochen grundsätzlich kein bzw. nur ein sehr geringer Kontakt notwendig ist und stattfindet. Lastenübertragungselemente sind die Schrauben, welche in der Platte verriegelt werden, in Verbindung mit der Platte selber. Das Plattenimplantat muss jedoch vergleichsweise nahe am Knochen platziert werden, weshalb derartige Implantate in der Regel anatomisch vorgeformt sind. Für das Setzen des Implantates bedarf es lediglich eines vergleichsweise kleinen Gewebeeinschnittes, so dass die Platte zwischen Knochen und Weichteile eingeschoben werden kann. Die Verriegelung erfolgt dann mittels weiterer, noch kleinerer Einschnitte über die Haut von aussen.

Kirschner-Drähte werden bei diesem System für die Grob fixierung und Verbindung des plattenähnlichen Implantates mit dem Kn ochen eingesetzt. Hierfür werden die Kirschner-Drähte eingebracht und später entnommen und durch entsprechende Verriegelungsschrauben ersetzt. Kirschner-Drähte sind zudem bei der Reposition der Fraktur nützlich. Nach der Reposition werden die Verriegelungsschrauben eingebracht und der Kirschner-Draht wird wieder entfernt.

Folglich ist von Bedeutung, dass ein Kirschner-Draht nicht nur in den Knochen eingebracht, sondern auch aus diesem wieder entfernt werden kann. Hierfür sind im Stand der Technik verschiedene Werkzeuge vorgesehen. So ist aus der US 6,015,413 eine Vorrichtung zum Extrahieren von Drähten, welche ein Gewinde aufweisen, offenbart. Die Vorrichtung ist zangenähnlich, so dass der Draht gefasst werden kann. Der Zug auf den Kirschner-Draht erfolgt durch ein mechanisches Auseinanderdrängen der beiden Zangenhälften, wodurch eine Verkürzung erzielt wird, die letztlich in eine Zugbewegung umgesetzt wird. Die Zugbewegung dieser Vorrichtung ist jedoch vergleichsweise grob. Eine Führung des Drahtes ist nicht gegeben. Der Kirschner-Draht kann abgebogen und verdreht werde n. Insgesamt ist folglich die Zugbewegung unbefriedigend.

Aus der DE-A1-44 18974 und der EP-A1-0 465 866 sind jeweils spezielle Drähte offenbart, für die dann jeweils ein spezielles Extraktionsmittel vorgesehen ist. So beschreibt die deutsche Offenlegungsechrift, aus der die Präambel der unabhängigen Ansprüche abgeleitet wird, einen Kompressionsgewindebohrdraht, über den eine Spannmutter aufgeschoben wird. Die Kompression des Drahtes wird durch Verdrehen der Spannmutter erzeugt. Die darin offenbarte Spannmutter ist nur für den in der Druckschrift offenbarten speziellen Draht einsetzbar. In der europäischen Patentanmeldung ist ein Draht für einen Fixateur zum Einrichten, Befestigen und Regulieren der Spannlage von Knochenabschnitten beschrieben. Der Draht ist sehr speziell ausgebildet und weist mehrere unterschiedliche Abschnitte auf. Auf einen besonderen Abschnitt dieses Drahtes ist ein so genanntes Haltemittel aufsetzbar, welches der Aufnahme des Bohrkopfes dient und somit eine Schutzfunktion erfüllt. Durch axiales Verschieben des Haltemittels kann der Draht schliesslich in seiner Verankerung gelöst werden. Eine Übertragung auf den Kirschner-Draht und dessen Entfernen, beispielsweise bei der Fixierung mittels LISS ist nicht möglich.

Weiter ist für die Verwendung in der LISS-Technik ein Zuginstrument bekannt. Hierbei wird ein speziell ausgebildeter Kirschner-Draht, der Zugdraht, in eine Führung eingesetzt. Der Zugdraht weist im vorderen Bereich eine Bohrspitze und ein Knochengewinde auf und im hinteren Bereich ein Regelgewinde. Auf diesem Regelgewinde ist eine Zug-Mutter vorgesehen, mit der die axiale Verschiebung des Tension Device bewirkt wird. Nachteilig ist jedoch, dass eine Limitierung in der Tiefe gegeben ist, durch die beiden unterschiedlichen Gewinde, die auf der Vorrichtung vorzusehen sind. Nachteilig ist ferner, dass die Zug-Mutter über eine grosse Distanz über das Regelgewinde bewegt werden muss, ehe die Führung erreicht ist, wo erst die Zugbewegung erfolgen kann.

Aufgabe der vorliegenden Erfindung ist daher, ein Repositionswerkzeug zum axialen Verschieben eines Kirschner-Drahtes bereitzustellen, mit dessen Hilfe ein Kirschner-Draht einfach, zuverlässig und möglichst exakt in axialer Richtung verschoben werden kann. Eine Begrenzung in der Tiefe soll möglichst nicht stattfinden. Ferner soll das Repositionswerkzeug möglichst universell einsetzbar sein.

Gelöst wird diese Aufgabe durch ein Repositionswerkzeug nach Anspruch 1 bzw. durch eine universell einsetzbare Mutter nach Anspruch 11 oder 15. Ausbildungen der Erfindung und Varianten dazu sind in den abhängigen Patentansprüchen angegeben.

Das erfindungsgemässe Repositionswerkzeug zum Verschieben eines Kirschner-Drahtes ist insbesondere zur Anwendung bei Knochenplatten, die zur Fixierung von Frakturen implantierbar sind, einsetzbar. Es weist einen Kirschner-Draht auf, der zumindest in einem Teilbereich ein Gewinde, insbesondere ein Knochengewinde hat. Das Knochengewinde kann beispielsweise ein Kortikalis- oder ein Spongiossgewindes sein. Ferner ist zumindest ein Führungsrohr vorgesehen, das über dem Kirschner-Draht positionierbar ist. Erfindungsgemäss ist eine Mutter vorgesehen, die geschlitzt ist und eine innere Bohrung aufweist, so dass die Mutter über dem Kirschner-Draht aufsetzbar und auf dem Führungsrohr abstrützbar ist.

Die Mutter ist folglich an einer Stelle geöffnet, so dass ein Durchgang zur zentralen Bohrung der Mutter entsteht. Sie weist einen durchgehenden Längsschlitz auf. Die Mutter wird seitlich aufgesetzt. Vorteilhaft weist die Mutter einen Querschlitz, einen ersten Längsteilschlitz und einen zweiten Langsteilschlitz auf, die miteinander verbunden sind. Die Mutter kann so durch Einfädeln auf den Draht aufgesetzt werden. Die Mutter ist so ähnlich stabil mit dem Draht verbunden, wie aus dem Stand der Technik bekannte Muttern, die auf den Draht aufgeschraubt und durch Drehbewegungen bis zum Führungsrohr bewegt werden müssen.

Die axiale Verschiebung des Kirschner-Drahtes wird durch eine Drehbewegung der Mutter erzielt. Durch die Drehbewegung ist die Position des Knochenfragmentes in axialer Richtung kontrollierbar. Vorzugsweise ist auf dem Kirschner-Draht ein durchgängiges Knochengewinde, insbesondere ein Kortikalis- oder Spongiossgewinde vorgesehen, was zahlreiche Vorteile mit sich bringt. Eine Tiefenilmitierung ist damit ausgeschlossen. Es bedarf ferner nicht des Vorsehens verschiedener Längen des Kirschner-Drahtes, sondern prinzipiell würde eine einzelne Länge genügen.

Gemäß einer ersten Ausführung wird die Mutter auf den Draht in der Art eines Clips aufgesetzt. Dies bedeutet, dass die beiden durch den Längsschlitz gebildeten Hälften der Mutter etwas auseinander gedrückt werden, so dass der Kirschner-Draht über diesen Längsschlitz in die mittige Bohrung hindurchführbar ist. In einer bevorzugten Ausführungsform ist auf der dem Längsschlitz gegenüber liegenden Innenseite der Mutter ein kleiner Einschnitt vorgesehen, der der verbleibenden Wand eine Scharnierfunktion zuteilt. Alternativ, wird dieses leichte Öffnen der Mutter durch ein vorzugsweise gefedertes Scharnier erleichtert.

Gemäß einer zweiten Ausführung erfolgt das Aufsetzen der Mutter durch ein bajonettartiges Einfädeln des Drahtes durch die Führungsschlitze der Mutter. Die Mutter weist hierfür einen Horizontal- oder Querschlitz und zwei teilweise ausgeführte Längsschlitze aus, als Längsteilschlitze bezeichnet. Der Draht wird durch Einführen in den Querschlitz und anschließende Drehbewegung, so dass er in die Längsteilschlitze eintritt, aufgefädelt.

Durch die erfindungsgemässe Gestaltung der Mutter kann diese an jeder beliebigen Stelle des Kirschner-Drahtes aufgesetzt werden. Ein Einfädeln vom Ende des Kirschner-Drahtes her, wie dies bei den Muttern im Stand der Technik notwendig ist, entfällt.

Die Mutter ist vorzugsweise aus Kunststoff gefertigt, insbesondere aus Polyethylen. Dadurch ist die Mutter billig herzustellen und kann für den einmaligen Gebrauch vorgesehen werden. Vorzugsweise wird die Mutter durch ein Sterilisationsverfahren, z.B. die im Klinikbetrieb übliche Dampfsterilisation, zerstört. Die Glastemperatur des verwendeten Kunststoffs liegt deshalb bevorzugt unter der Sterilisationstemperatur, üblicherweise ca. 150 °C im Klinikbereich. Dadurch wird nicht nur erzielt, dass die Mutter nur einmal verwendet wird, sondern dass auch der Kirschner-Draht nur einmal verwendet werden kann. Wenn die Mutter im Dampfsterilisationsverfahren etwas schmilz, so verklebt sie mit dem Draht, wodurch dieser ebenfalls nicht weiter verwendet werden kann. So wird sichergestellt, dass nicht nur die Mutter sondern auch der Kirschner-Draht nur einmal verwendet werden können, was insbesondere wegen der schlecht zugänglichen Räume zwischen Draht und Mutter von Vorteil ist. Das Repositionswerkzeug kann ferner für den Einmalgebrauch in einer sterilen Verpackung, fertig für den Gebrauch, ausgeliefert werden.

Weiterhin kann die innere Bohrung der Mutter ein Gewinde aufweisen. Es kann sich hierbei ebenfalls um ein Knochengewinde handeln oder aber auch um ein symmetrisches Gewinde, in der Art eines metrischen Gewindes. Das symmetrische Gewinde hat dabei den Vorteil, dass die Mutter in jeder beliebigen Richtung auf den Kirschner-Draht aufgesetzt werden kann; es weist dann keine Vorzugsrichtung auf. Im Gegensatz dazu hat ein sägezahnförmiges Knochengewinde eine Vorzugsrichtung, so dass beim Aufsetzen der Mutter darauf geachtet werden muss, dass dieses gewahrt wird. Grundsätzlich bedarf es jedoch überhaupt keines Gewindes, da das Knochengewinde des Kirschner-Drahtes sich in der inneren Bohrung der Mutter durch die Drehbewegung selbst ein Gewinde ausbilden kann.

Die Zeichnungen werden nachfolgend mit Bezugnahme auf ein PHILOS ®-Plattensystem (Proximal Humerus Internal Locking System) von Synthes ® beschrieben. Die Erfindung ist jedoch nicht darauf eingeschränkt, sondern steht vielmehr auch anderen Osteosynthesebereichen offen, in denen ein Kirschner-Draht mit Knochengewinde oder dergleichen zum Einsatz kommt und wobei dieser wieder entfernt werden muss, insbesondere bei einer LISS-Fixierung. Auch bei anderen Fixierungstechniken von Frakturen findet das Repositionswerkzeug folglich Anwendung. Die Patentansprüche sind dementsprechend breit auszulegen.

PHILOS ® wird insbesondere bei dislozierten Fragmentfrakturen des proximalen Humerus eingesetzt. Ähnlich LISS, das bei Frakturen der proximalen lateralen Tibia und des distalen Femur eingesetzt wird, ist ein anatomisch vorgeformtes Implantat vorgesehen , das in Verbindung mit den Verriegelungsschrauben die Last trägt.

Die Bezugszeichenliste und die Zeichnungen sind zusammen mit den in den Patentansprüchen beschriebenen bzw. geschützten Gegenständen integrierender Bestandteil der Offenbarung dieser Anmeldung.

### Figurenbeschreibung

Die Figuren werden zusammenhängend und übergreifend beschrieben. Gleiche Bezugszeichen bedeuten gleiche Bauteile; Bezugszeichen mit unterschiedlichen Indizes geben funktionengleiche Bauteile an.

Es zeigen dabei:
- Fig. 1:: das erfindungsgemässe Repositionswerkzeug in erfindungsgemässer Verwendung in schematischer perspektivischer Darstellung;
- Fig. 2:: ein erfindungsgemässes Repositionswerkzeug in schematischer Aufsicht;
- Fig. 3:: eine Mutter des Repositionswerkzeuges in Aufsicht (A) und im Längsschnitt entlang der Linie A-A;
- Fig. 4:: ein weiteres Ausführungsbeispiel einer Mutter in Aufsicht; und
- Fig. 5:: noch ein weiteres Ausführungsbeispiel einer Mutter im Längsschnitt (A), in Seitenansicht (B) und in Aufsicht (C).

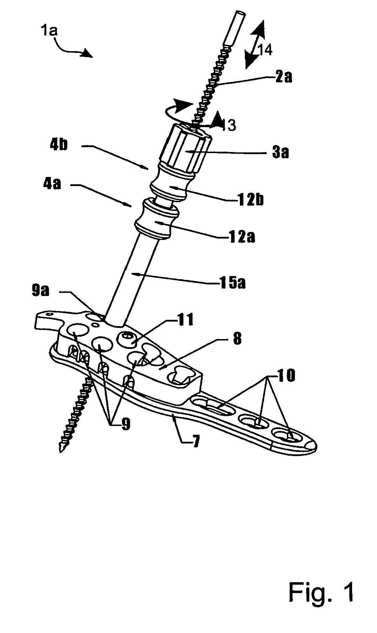

Fig. 1 zeigt das Repositionswerkzeug 1a in seiner bestimmungsgemässen Verwendung. Es ist in eine Bohrführung 9a eines Zielgerätes 8 eingebracht. Das Zielgerät 8 ist mit Hilfe einer Schraube 11 auf dem Implantat 7 befestigt. Das Implantat 7 ist plattenförmig und weist mehrere Bohrungen 10 auf, durch die Verriegelungsschrauben hindurchführbar sind und mit deren Hilfe die Fixierung des Knochens durchgeführt wird. Das Implantat 7 verbleibt zumindest bis zur Ausheilung der Fraktur im Körper. Anders das Zielgerät 8, welches nur temporär auf das Implantat 7 aufgesetzt wird. Das Zielgerät 8 weist zahlreiche Bohrführungen 9 auf. Mit Hilfe der Bohrführungen 9 werden die Verriegelungsschrauben eingebracht. Das Zielgerät dient hierbei als Führung und Positionierungshilfe. Wie aus der Fig. 1 ersichtlich wird, ist das Repositionswerkzeug 1a in eine solche Bohrführung 9a eingebracht.

Das Repositionswerkzeug 1 a weist einen Kirschner-Draht 2a auf. Vorzugsweise ist der Kirschner-Draht 2a aus Stahl gefertigt. Dieser ist durch zumindest ein Führungsrohr 4 hindurchgeführt. Im Ausführungsbeispiel der Fig. 1 sind zwei Führungsrohre 4a, 4b vorgesehen. Die Führungsrohre 4 sind hülsenartig gestaltet. Sie weisen jeweils einen Schaft 15 und einen Griff 12 auf. Die Führungsrohre sind konzentrisch und werden ineinander eingeführt, so dass lediglich jeweils der Griffbereich 12a, 12b zu sehen ist. Nur vom Führungsrohr 4a ist auch der Schaft 15a in seiner gesamten Länge zu erkennen. Die Führungsrohre 4 üben unterschiedliche Funktionen aus. So handelt es sich beispielsweise bei dem Führungsrohr 4a um die so genannte Gewebeschutzhülse und bei dem Führungsrohr 4b um die so genannte Bohrbüchse. Die Gewebeschutzhülse dient beispielsweise auch als Einführhülse für eine Verriegelungsschraube und ausserdem auch für das Aufnehmen einer Bohrbüchse, dem Führungsrohr 4b. Beim PHILOS ®-System ist in der Bohrbüchse, des heisst in dem Führungsrohr 4b, zusätzlich auch der Kirschner-Draht gemäss der Erfindung geführt. Prinzipiell genügt es jedoch für die Ausführung der Erfindung, wenn ein einzelnes Führungsrohr4 vorgesehen ist. Das Führungsrohr 4 wird in die Bohrführung 9a eingebracht. Der Kirschner-Draht 2a ist nun durch das Führungsrohr 4 hindurch einbringbar und in diesem axial verschiebbar.

Zum Herausziehen des Kirschner-Drahtes 2a wird eine erfindungsgemässe Mutter 3a seitlich über ihren Schiltz 16 aufgesetzt. Diese Mutter 3a stützt sich in Gebrauchslage auf dem Führungsrohr 4 ab. Im Ausführungsbeispiel der Fig. 1 stützt sich die Mutter auf dem Griff 12b des Führungsrohres 4b ab. Durch eine Drehbewegung der Mutter 3a, angedeutet durch den Pfeil 13, wird der Kirschner-Draht 2a in axialer Richtung verschoben, angedeutet durch den Pfeil 14 Die axiale Verschiebung eines durch den Kirschner-Draht bewegten Knochenfragmentes erfolgt in Richtung zum Implantat 7. Nach dem Entfernen des Kirschner-Drahtes 2a kann an seine Stelle eine Verriegelungsschraube o.dgl. gesetzt werden.

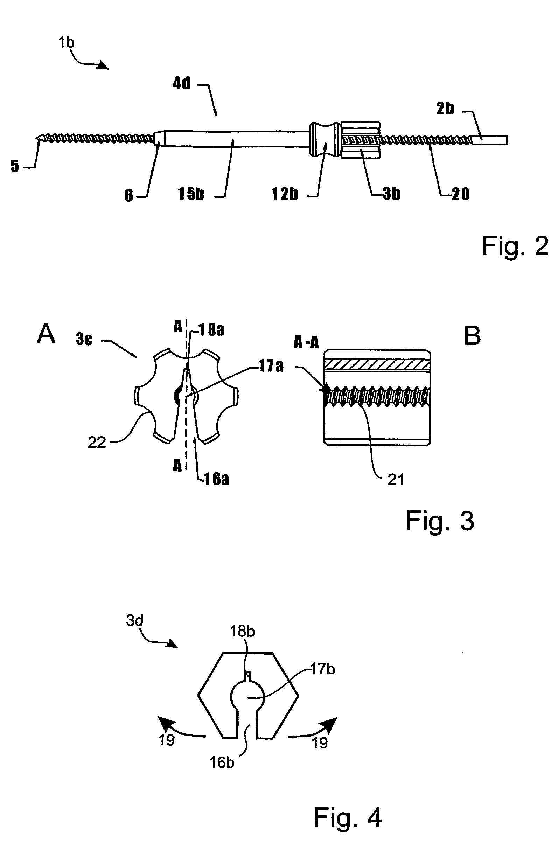

Fig. 2 zeigt schematisch ein erfindungsgemässes Repositionsvverkzeug 1b. Der Kirschner-Draht 2b weist durchgängig ein Gewinde auf, im Ausführungsbeispiel der Fig. 2 ein Kortikalisgewinde 20. An se Iner dem Knochen zugewandten Ende ist eine Bohrspitze 5 vorgesehen. Der Kirschner-Draht 2b ist durch ein Führungsrohr 4d hindurchgeführt. Das Führungsrohr 4d weist einen Schaft 15b auf, der von dem einen Ende von dem Griff 12b begrenzt wird. Am anderen Ende kann eine Abschrägung 6 vorgesehen sein, die an eine entsprechende Ausnehmung des Implantates 7 bzw. des Zielgerätes 8 angepasst ist. Der Griff 12b dient überwiegend zum Festhalten des Führungsrohres 4 und zu dessen Betätigung. Auf den Kirschner-Draht 2b ist wiederum eine Mutter 3b aufgesetzt.

Die Fig. 3 und 4 zeigen je ein Ausführungsbeispiel einer Mutter 3c bzw. 3d. In Fig. 3 ist sowohl eine Aufsicht (A) als auch ein Längsschnitt (B) entlang der gestrichelten Linie A-A in Fig. 3A dargestellt. Fig. 4 zeigt eine schematische Aufsicht auf die Mutter 3d. Erfindungsgemäss ist ein Längsschlitz 16a, 16b vorgesehen. Damit ist ein Durchtritt zur inneren Bohrung 17a, 17b geschaffen. Der Kirschner-Draht 2 kann nun durch diesen Längsschlitz 16a, 16b hindurchgeschoben werden und befindet sich dann in der inneren Bohrung 17a, 17b. Die Mutter kann ein Innengewinde aufweisen, insbesondere ein Knochengewinde oder ein metrisches Gewinde. In Fig. 3B ist ein symmetrisches Gewinde 21 gezeigt. Gegenüber liegend des Längsschlitzes 16 ist sowohl im Ausführungsbeispiel der Fig. 3 als auch in dem der Fig. 4 eine Ausnehmung 18a bzw. 18b vorgesehen. Sie hat allgemein die Form eines Einschnittes oder Schlitzes. Aus einem Vergleich der Fig. 3 und 4 wird deutlich, dass die Gestalt der Ausnehmung 18 variieren kann. Die Ausnehmung 18 hat eine Scharnierfunktion, so dass die Mutter 3c, 3d im Bereich des Längsschlitzes 16a, 16b gespreizt werden kann, angedeutet durch die Pfeile 19 in Fig. 4. Das Spreizen erleichtert das Aufsetzen der Mutter 3c, 3d auf den Kirschner-Draht 2. Aus einem Vergleich der Fig. 3 und 4 ergibt sich weiter, dass der Längsschlitz 16 verschieden gestaltet sein kann. In Fig. 3 ist er abgeschrägt, so dass das seitliche Einführen der Mutter 3c auf den Kirschner-Draht 2 erleichtert wird.

Die Mutter 3c weist überdies Griffmulden 22 auf, die das Angreifen der Mutter 3c und damit deren Drehbewegung erleichtern. Die Ausgestaltung der Hülsen ist jedoch nicht einschränkend für die erfinderische Neuerung.

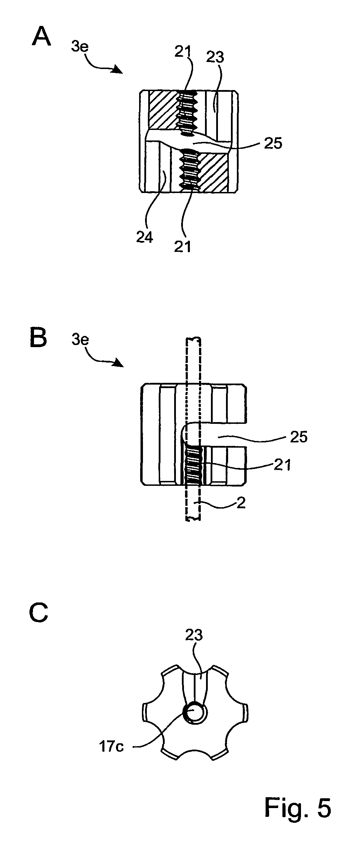

Die Fig. 5 zeigt ein weiteres Ausführungsbeispiel einer Mutter 3e. Es sind sowohl eine Aufsicht (C) als auch ein Längsschnitt (A) und eine Seitenansicht dargestellt. Die Mutter 3e weist wiederum eine innere Bohrung 17c auf, welche ein Gewinde 21 haben kann. Weiterhin weist sie einen Querschlitz 25 und einen ersten und einen zweiten Längsteilschlitz 23, 24 auf. Wie insbesondere aus der Fig. 5A ersichtlich ist, stehen diese Schlitze miteinander in Verbindung. Ferner sind alle Schlitze bis zur inneren Bohrung 17c geführt. Dadurch wird wiederum ein Durchtritt für den Kirschner-Draht 2 geschaffen. Dieser wird bajonettartig eingefädelt: Die Mutter 3e wird hierfür zunächst mit ihrem Querschlitz 25 auf den Draht aufgesetzt. Das erste Aufsetzen erfolgt somit um 90° versetzt im Vergleich zu der Mutter 3a-3d, die in den Fig. 1-4 dargestellt ist. Dort wird die Mutter 3a-3d parallel zur Längsachse und damit zu der inneren Bohrung 17 aufgesetzt. Bei dem Ausführungsbeispiel der Fig. 5 wird die Mutter 3e nicht parallel zu der inneren Bohrung 17 aufgesetzt, sondern im Wesentlichen senkrecht dazu. Anschließend wird die Mutter 3e um ca. 90° gedreht, so dass der Kirschner-Draht 2 jetzt in die Längsteilschlitze 23, 24 eintritt. In Fig. 5B wurde ein Kirschner-Draht 2 teilweise schematisch eingetragen. Die gestrichelte Linie stellt die Bereiche dar, die in der Darstellung sichtbar sind, die punktierte Linie jener Bereich des Drahtes, der nicht zu sehen ist. Die Mutter 3e ist durch diese erfindungsgemäße Gestaltung auf dem Kirschner-Draht 2 sicher geführt.

Der Querschlitz 25 kann insbesondere annähernd mittig angeordnet sein. Dann handelt es sich bei den Längsteilschlitzen 23, 24 um Längshalbschlitze. Diese Aufteilung kann jedoch auch anders gewählt werden, was für den Fachmann ohne Weiteres ersichtlich ist.

### Bezugszeichenliste

- 1: Repositionswerkzeug
- 2: Kirschner-Draht
- 3: Mutter
- 4: Führungsrohr
- 5: Bohrspitze
- 6: Abschrägung
- 7: Implantat
- 8: Zielgerät
- 9: Bohrführung
- 10: Bohrung
- 11: Schraube
- 12: Griff
- 13,14,19: Pfeil
- 15: Schaft
- 16: Längsschlitz
- 17: innere Bohrung
- 18: Ausnehmung
- 20: Knochengewinde
- 21: Gewinde
- 22: Griffmulde
- 23: erster Längsteilschlitz
- 24: zweiter Längsteilschlitz
- 25: Querschlitz

## Patentansprüche

1. Repositionswerkzeug (1) zum axialen Verschieben eines Kirschner-Drahtes (2), insbesondere zur Anwendung bei Knochenplatten, die zur Fixierung von Frakturen implantierbar sind, aufweisend: einen Kirschner-Draht, der zumindest in einem Teilbereich ein Gewinde (20) aufweist, und zumindest ein Führungsrohr (4), das über dem Kirschner-Draht positionierbar ist, **dadurch gekennzeichnet, dass** eine Mutter (3) vorgesehen ist, die einen Längsschlitz (16) und eine innere Bohrung (17) aufweist, so dass die Mutter (3) seitlich auf dem Kirschner-Draht (2) aufsetzbar und auf dem Führungsrohr (4) abstützbar ist, wobei der Kirschner-Draht (2) durch Drehbewegung der Mutter (3) in axialer Richtung (14) in dem Führungsrohr (4) sowie relativ zur Mutter (3) und dem Führungsrohr (4) verschiebbar ist.

2. Repositionswerkzeug nach Anspruch 1, **dadurch gekennzeichnet, dass** die Mutter (3) einen Querschlitz (25), einen ersten Längstellschliltz (23) und einen zweiten Längsteilschlitz (24) aufweist, die miteinander verbunden sind.

3. Repositionswerkzeug nach einem der vorhergehenden Ansprüche, **dadurch gekennzeichnet dass** die Mutter (3) aus Kunststoff, insbesondere aus Polyethylen gefertigt ist.

4. Repositionswerkzeug nach einem der vorhergehenden Ansprüche, **dadurch gekennzeichnet, dass** das Gewinde (20) des Kirschner-Drahtes (2) ein Knochengewinde, insbesondere ein Kortikalis- oder Spongiossgewinde ist.

5. Repositionswerkzeug nach einem der vorhergehenden Ansprüche, **dadurch gekennzeichnet dass** die Mutter (3) ein innengewinde (21) aufweist, insbesondere ein Knochengewinde oder ein symmetrisches Gewinde.

6. Repositionswerkzeug nach einem der vorhergehenden Ansprüche, **dadurch gekennzeichnet, dass** der Kirschner-Draht (2) im Wesentlichen über seine gesamte Länge ein durchgängiges Knochengewinde (20) aufweist.

7. Repositionswerkzeug nach einem der vorhergehenden Ansprüche, **dadurch gekennzeichnet, dass** die Mutter auf der dem Längsschlitz (16) gegenüber liegenden Seite der Bohrung (17) eine nutförmige Ausnehmung (18) aufweist.

8. Repositionswerkzeug nach einem der Ansprüche 1 bis 7, **dadurch gekennzeichnet, dass** die Mutter (3) auf der dem Längsschlitz (16) gegenüber liegenden Seite der Bohrung (17) ein Scharnier aufweist bzw. dass die dem Längsschlitz (16) gegenüberliegende Wand der Mutter (3) als Scharnier ausgebildet ist.

9. Repositionswerkzeug nach einem der vorhergehenden Ansprüche, **dadurch gekennzeichnet, dass** die Mutter (3) durch ein Sterilisationsverfahren, insbesondere durch Dampfsterilisation, zerstörbar ist.

10. Repositionswerkzeug nach einem der vorhergehenden Ansprüche, **dadurch gekennzeichnet, dass** dieses in Verbindung mit Osteosynthessplatten, insbesondere PHILOS®-Platten od er LISS-Platten verwendbar ist.

11. Mutter für ein Repositionswerkzeug nach einem der vorhergehenden Ansprüche, **dadurch gekennzeichnet, dass** sie einen seitlichen Längsschlitz (16) und eine innere Bohrung (17) aufweist, so dass die Mutter (3) auf einen Kirschner-Draht (2) seitlich über den Längsschlitz (16) aufsetzbar ist, wobei die dem Längsschlitz (16) gegenüberliegende Wand der Mutter (3) als Scharnier ausgebildet ist.

12. Mutter nach Anspruch 11, **dadurch gekennzeichnet, dass** die Mutter auf der dem Längsschlitz (16) gegenüber liegenden Seite der Bohrung (17) eine nutförmige Ausnehmung (18) aufweist.

13. Mutter nach Anspruch 11 oder 12, **dadurch gekennzeichnet, dass** sie aus Kunststoff, insbesondere aus Polyethylen gefertigt und durchinsbesondere thermisches - Sterilisierverfahren zerstörbar ist.

14. Mutter nach einem der vorhergehenden Ansprüche 12 bis 14, **dadurch gekennzeichnet, dass** sie in ihrer Bohrung ein innengewinde (21) aufweist, insbesondere ein Knochengewinde oder ein symmetrisches Gewinde.

15. Mutter für ein Repositionswerkzeug nach einem der vorhergehenden Ansprüche, **dadurch gekennzeichnet, dass** eine Mutter (3) vorgesehen ist, die einen Querschlitz (25), einen ersten Längstellschlitz (23) und einen zweiten Längstellschlitz (24) und eine innere Bohrung (17) aufweist, die miteinander verbunden sind.

## Claims

1. Repositioning tool (1) for axially displacing a Kirschner wire (2), in particular for use in bone plates which can be implanted for fixing fractures, having: a Kirschner wire having at least in one partial area a thread (20), and at least one guide tube (4) that can be positioned above the Kirschner wire, **characterized in that** a nut (3) is provided having a longitudinal slot (16) and an internal bore (17), so that the nut (3) can be placed laterally on the Kirschner wire (2) and rested on the guide tube (4), with the Kirschner wire (2) being displaceable through rotational movement of the nut (3) in axial direction (14) in the guide tube (4) as well as relative to the nut (3) and the guide tube (4).

2. The repositioning tool according to claim 1, **characterized in that** the nut (3) has a transverse slot (25), a first longitudinal partial slot (23) and a second longitudinal partial slot (24) which are connected with each other.

3. The repositioning tool according to any of the preceding claims, **characterized in that** the nut (3) is made of plastic, in particular polyethylene.

4. The repositioning tool according to any of the preceding claims, **characterized in that** the thread (20) of the Kirschner wire (2) is a bone thread, in particular a corticalis thread or spongiosa thread.

5. The repositioning tool according to any of the preceding claims, **characterized in that** the nut (3) has an internal thread (21), in particular a bone thread or a symmetrical thread.

6. The repositioning tool according to any of the preceding claims, **characterized in that** the Kirschner wire (2) essentially has a bone thread (20) extending continuously over its complete length.

7. The repositioning tool according to any of the preceding claims, **characterized in that** the nut has a groove-shaped gap (18) on the side of the bore (17) opposite the longitudinal slot (16).

8. The repositioning tool according to any of claims 1 to 7, **characterized in that** the nut (3) has a hinge on the side of the bore (17) opposite the longitudinal slot (16) or that the wall of the nut (3) opposite the longitudinal slot (16) is configured as a hinge.

9. The repositioning tool according to any of the preceding claims, **characterized in that** the nut (3) can be destroyed through a sterilization process, in particular vapor sterilization.

10. The repositioning tool according to any of the preceding claims, **characterized in that** it can be used in connection with osteosynthesis plates, in particular PHILOS^{®} plates or LISS plates.

11. A nut for a repositioning tool according to any of the preceding claims, **characterized in that** it has a lateral longitudinal slot (16) and an internal bore (17), so that the nut (3) can be placed on a Kirschner wire (2) laterally via the longitudinal slot (16), with the wall of the nut (3) opposite the longitudinal slot (16) being configured as a hinge.

12. The nut according to claim 11, **characterized in that** the nut has a groove-shaped gap (18) on the side of the bore (17) opposite the longitudinal slot (16).

13. The nut according to claim 11 or 12, **characterized in that** it is made of plastic, in particular polyethylene, and can be destroyed through a - particularly thermal - sterilization process.

14. The nut according to any of the preceding claims 12 to 14, **characterized in that** it has in its bore an internal thread (21), in particular a bone thread or a symmetrical thread.

15. A nut for a repositioning tool according to any of the preceding claims, **characterized in that** a nut (3) is provided which has a transverse slot (25), a first longitudinal partial slot (23) and a second longitudinal partial slot (24) and an internal bore (17) which are connected with each other.

## Revendications

1. Outil de repositionnement (1) destiné au déplacement axial d'un fil de Kirschner (2), destiné en particulier à être utilisé avec des plaques d'ostéosynthèse pouvant être implantées pour la fixation de fractures, comprenant : un fil de Kirschner comprenant au moins dans une zone partielle un filetage (20), et au moins un tube de guidage (4) pouvant être positionné au-dessus du fil de Kirschner, **caractérisé en ce qu'**un écrou (3) comprend une fente longitudinale (16) et un trou intérieur (17), de sorte que l'écrou (3) peut être posé latéralement sur le fil de Kirschner (2) et peut s'appuyer sur le tube de guidage (4), le fil de Kirschner (2) pouvant être déplacé dans le tube de guidage (4) ainsi que par rapport à l'écrou (3) et au tube de guidage (4) dans la direction axiale (14) grâce au déplacement rotatif de l'écrou (3).

2. Outil de repositionnement selon la revendication 1, **caractérisé en ce que** l'écrou (3) comprend une fente transversale (25), une première fente partielle longitudinale (23) et une seconde fente partielle longitudinale (24) reliées les unes aux autres.

3. Outil de repositionnement selon l'une quelconque des revendications précédentes, **caractérisé en ce que** l'écrou (3) est fabriqué en matière plastique, en particulier en polyéthylène.

4. Outil de repositionnement selon l'une quelconque des revendications précédentes, **caractérisé en ce que** le filetage (20) du fil de Kirschner (2) est un filetage osseux, en particulier un filetage cortical ou un filetage spongieux.

5. Outil de repositionnement selon l'une quelconque des revendications précédentes, **caractérisé en ce que** l'écrou (3) comprend un filetage intérieur (21), en particulier un filetage osseux ou un filetage symétrique.

6. Outil de repositionnement selon l'une quelconque des revendications précédentes, **caractérisé en ce que** le fil de Kirschner (2) comprend un filetage osseux (20) continu sensiblement sur toute sa longueur.

7. Outil de repositionnement selon l'une quelconque des revendications précédentes, **caractérisé en ce que** l'écrou comprend sur le côté du trou (17) opposé à la fente longitudinale (16) un évidement (18) en forme de rainure.

8. Outil de repositionnement selon l'une quelconque des revendications 1 à 7, **caractérisé en ce que** l'écrou (3) sur le côté du trou (17) opposé à la fente longitudinale (16) comprend une charnière, ou **en ce que** la paroi de l'écrou (3) opposée à la fente longitudinale (16) est conçue comme une charnière.

9. Outil de repositionnement selon l'une quelconque des revendications précédentes, **caractérisé en ce que** l'écrou (3) peut être détruit par un processus de stérilisation, en particulier par stérilisation à la vapeur.

10. Outil de repositionnement selon l'une quelconque des revendications précédentes, **caractérisé en ce qu'**il peut être utilisé en association avec des plaques d'ostéosynthèse, en particulier des plaques PHILOS^{®} ou des plaques LISS.

11. Ecrou pour un outil de repositionnement selon l'une quelconque des revendications précédentes, **caractérisé en ce qu'**il comprend une fente longitudinale latérale (16) et un trou intérieur (17), de sorte que l'écrou (3) peut être posé latéralement sur un fil de Kirschner (2) au-dessus de la fente longitudinale (16), la paroi de l'écrou (3) opposée à la fente longitudinale (16) étant conçue comme une charnière.

12. Ecrou selon la revendication 11, **caractérisé en ce que** l'écrou comprend sur le côté du trou (17) opposé à la fente longitudinale (16) un évidement (18) en forme de rainure.

13. Ecrou selon la revendication 11 ou 12, **caractérisé en ce qu'**il est fabriqué en matière plastique, en particulier en polyéthylène et peut être détruit par un processus de stérilisation, en particulier un processus de stérilisation thermique.

14. Ecrou selon l'une quelconque des revendications 12 à 13, **caractérisé en ce qu'**il comprend dans son trou un filetage intérieur (21), en particulier un filetage osseux ou un filetage symétrique.

15. Ecrou pour un outil de repositionnement selon l'une quelconque des revendications précédentes, **caractérisé en ce qu'**un écrou (3) comprend une fente transversale (25), une première fente partielle longitudinale (23) et une seconde fente partielle longitudinale (24) et un trou intérieur (17) reliés les uns aux autres.
